(19) Europäisches Patentamt

European Patent Office

Office européen des brevets

(11) EP 0 755 672 B1

(12) FASCICULE DE BREVET EUROPEEN

(45) Date de publication et mention
de la délivrance du brevet:
**03.09.1997 Bulletin 1997/36**

(51) Int. Cl.⁶: **A61K 7/48**

(21) Numéro de dépôt: **96401382.5**

(22) Date de dépôt: **21.06.1996**

(54) **Composition siliconée contenant un actif sensible à l'eau**

Silizium-Zusammensetzung, die ein wasserempfindliche Wirkstoff enthält

Silicon composition containing a water-sensitive agent

(84) Etats contractants désignés:
**DE ES FR GB IT**

(30) Priorité: **25.07.1995 FR 9509028**

(43) Date de publication de la demande:
**29.01.1997 Bulletin 1997/05**

(73) Titulaire: **L'OREAL**
**75008 Paris (FR)**

(72) Inventeurs:
• **Afriat, Isabelle**
**75014 Paris (FR)**
• **Gagnebien, Didier**
**92320 Chatillon (FR)**
• **Pecile, Isabelle**
**92160 Antony (FR)**

(74) Mandataire: **Andral, Christophe André Louis et al**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 330 369**      **EP-A- 0 516 547**
**EP-A- 0 611 565**

• **PATENT ABSTRACTS OF JAPAN vol. 7, no. 151
(C-174) [1296] , 2 Juillet 1983 & JP-A-58 063750
(ASAHI DENKA KOGYO K.K.), 15 Avril 1983,**
• **CHEMICAL ABSTRACTS, vol. 111, no. 6, 7 Août
1989 Columbus, Ohio, US; abstract no. 45032p,
page 367; XP002016520 & JP-A-01 043 342
(SHISEIDO CO.,LTD) 15 Février 1989**

**Description**

La présente invention a pour objet un milieu stable, compatible en particulier avec une application topique susceptible de contenir un actif sensible à l'eau. La composition ainsi obtenue est plus spécialement destinée à être utilisée dans les domaines cosmétique et/ou dermatologique pour nettoyer et/ou soigner et/ou protéger la peau et/ou les fibres kératiniques.

Il est connu d'introduire dans les compositions cosmétiques et/ou dermatologiques des actifs en vue d'apporter des traitements spécifiques à la peau et/ou aux cheveux, par exemple pour nettoyer la peau, pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire ou pour traiter la séborrhée des cheveux.

Par exemple, l'acide ascorbique (ou vitamine C) est connu pour stimuler la croissance du tissu conjonctif, et notamment celle du collagène. Il permet aussi de renforcer les défenses du tissu cutané contre les agressions extérieures telles que le rayonnement ultraviolet ou la pollution. Il est également utilisé pour enlever les taches et la pigmentation de la peau, et aussi pour favoriser la cicatrisation de la peau.

Il est aussi connu d'introduire dans les compositions cosmétiques des enzymes, et notamment des protéases utilisées pour leurs propriétés protéolytiques. Ces enzymes sont recherchées dans le domaine cosmétique pour leur pouvoir lissant et nettoyant, et leur aptitude à éliminer les cellules mortes de la peau.

Malheureusement, ces actifs ainsi que d'autres, présentent l'inconvénient d'être instables en milieu aqueux et d'être facilement dégradés ou modifiés sous l'influence de l'eau. Ils perdent ainsi rapidement de leur activité au cours du temps et cette instabilité va à l'encontre de l'efficacité recherchée.

Aussi, différents moyens ont été envisagés pour pallier à cet inconvénient. En particulier, il a été envisagé de mettre un actif, notamment une enzyme dans une composition pulvérulente (voir le document JP-A-63-130514). D'ailleurs, la plupart des produits de nettoyage de la peau contenant une enzyme se présentent sous cette forme. Il a été aussi envisagé d'utiliser ces actifs, et notamment les enzymes, sous forme immobilisée sur des supports polymériques (voir le document JP-A-61-207499) ou dans des microcapsules (voir le document JP-A-61-254244). Malheureusement, certains de ces moyens nécessitent une mise en oeuvre particulière, ce qui accroît le coût et le temps de préparation de la composition.

Une autre solution consiste à les incorporer dans un milieu liquide anhydre (voir le document US-A-5322683). Malheureusement, cette solution limite la forme galénique de la composition et ne permet pas l'incorporation d'actifs hydrophiles.

Il subsiste donc le besoin d'un milieu stable susceptible de contenir des actifs cosmétiques et/ou dermatologiques hydrophiles et/ou lipophiles sensibles à l'eau, dans lequel ces derniers conserveraient toutes leurs propriétés et donc leur efficacité au cours du temps.

La demanderesse a maintenant trouvé de manière inattendue qu'une émulsion huile-dans-eau, dont la phase huileuse contient une grande quantité d'huile siliconée, en présence d'un ou de plusieurs polyols en une quantité efficace pour obtenir une valeur d'activité en eau de l'émulsion, inférieure ou égale à 0,85, était susceptible de maintenir l'activité d'un actif sensible à l'eau et d'éviter la dégradation de l'actif.

Aussi, la présente invention a pour objet une émulsion comprenant une phase huileuse dispersée dans une phase aqueuse à l'aide d'un agent émulsionnant siliconé, dont la phase huileuse comporte au moins 50 % en poids d'huile siliconée, caractérisée en ce que la phase aqueuse contient au moins un polyol en une quantité efficace pour obtenir une valeur d'activité en eau de l'émulsion, inférieure ou égale à 0,85.

Pour une application dans les domaines cosmétique et/ou dermatologique, ces polyols peuvent jouer le rôle d'actif notamment hydratant.

De façon avantageuse, cette émulsion peut être utilisée comme support d'actif sensible à l'eau. Aussi, l'invention a aussi pour objet une composition, caractérisée en ce qu'elle contient au moins un actif à action topique sensible à l'eau et une émulsion telle que définie ci-dessus.

Certes, il est connu d'utiliser dans les domaines cosmétique et dermatologique des émulsions huile-dans-eau dont la phase huileuse contient une forte proportion d'huile siliconée. Ainsi, le document EP-A-516547 divulgue des compositions de ce type, mais il n'enseigne pas l'introduction d'une grande quantité de polyol, notamment en vue de stabiliser les actifs sensibles à l'eau.

Par ailleurs, il est connu que la teneur en eau peut avoir une influence sur la stabilité des actifs sensibles à l'eau. Ainsi, le document de D. Tzanos (Behavior of enzymes by controlling the medium water activity ; Riv. Ital. Essenze, Profumi, Piante Off., Aromi, Saponi, Cosmet., Aerosol, 1977, vol.59, n°5, pages 208-211) incite l'homme du métier à utiliser des tensioactifs pour la stabilisation des enzymes en milieu aqueux ou à fixer les enzymes sur un support poreux. En revanche, il écarte l'homme du métier d'utiliser des glycols et il ne suggère pas d'utiliser un milieu siliconé.

Or, il a été maintenant trouvé que l'on peut éviter la dégradation de tels actifs en les introduisant dans l'émulsion huile-dans-eau selon l'invention.

Selon une forme préférée de réalisation de l'invention, la quantité du ou des polyols doit être telle que la valeur d'activité en eau de l'émulsion est inférieure ou égale à 0,7.

L'activité en eau $a_w$ d'un milieu contenant de l'eau est le rapport de la pression de vapeur d'eau du produit 《$P_{H2O}$ produit 》et de la pression de vapeur de l'eau pure 《$P_{H2O}$ pur 》à la même température. Elle peut être exprimée aussi comme le rapport du nombre de molécules d'eau 《$N_{H2O}$ 》sur le nombre de molécules totales 《$N_{H2O}$ + $N_{corps\ dissous}$ 》 qui tient compte de celles des corps dissous 《 $N_{corps\ dissous}$ 》

Elle est donnée par les formules suivantes :

$$a_w = \frac{P_{H2O}\,produit}{P_{H2O}\,pur} = \frac{N_{H2O}}{N_{H2O} + N_{corps\ dissous}}$$

On peut utiliser différentes méthodes pour mesurer l'activité en eau. La plus courante est la méthode manométrique par laquelle on mesure directement la pression de vapeur.

De manière classique, une composition cosmétique ou dermatologique a une activité en eau située autour de 0,9 à 0,96. Une activité en eau inférieure à 0,85 représente une diminution notable de l'activité en eau.

Le polyol utilisé selon l'invention peut être notamment choisi parmi la glycérine et les glycols, en particulier le propylène glycol et les polyéthylène glycols.

Selon l'invention, la phase aqueuse de l'émulsion contenant le polyol peut représenter de 60 à 90 %, et de préférence de 70 à 85 % en poids par rapport au poids total de l'émulsion. Dans cette phase aqueuse, le ou les polyols utilisés selon l'invention sont de préférence présents en une quantité allant d'au moins 30 % en poids, de préférence de 40 à 90 % en poids, et mieux de 60 à 85 % en poids par rapport au poids total de l'émulsion.

Selon un mode de réalisation préféré de l'invention, le ou les polyols se trouvent totalement ou partiellement sous forme complexée avec un polymère acrylique ou méthacrylique. Le polymère peut également comprendre de l'eau liée, c'est-à-dire être complexé avec un mélange d'eau et de polyol(s).

On entend par polymère acrylique ou méthacrylique un homopolymère ou un copolymère d'acide acrylique ou méthacrylique ou un homopolymère ou un copolymère d'un dérivé d'acide acrylique ou méthacrylique.

La quantité de tels polymères avec le ou les polyols et éventuellement l'eau complexés, dans l'émulsion selon l'invention va de préférence de 60 à 90 % en poids, et mieux de 70 à 85 % en poids par rapport au poids total de l'émulsion.

On peut citer comme homopolymère complexant l'eau et les polyols ceux vendus sous les dénominations de Norgel et de Lubrajel CG par la société Guardian. Ces polymères sont des polyacrylates de glycéryle complexés avec plus de 65 % de glycérine et/ou de propylène glycol et moins de 35 % en poids d'eau liée. Ces polymères apportent le polyol et l'eau complexés, et éventuellement jouent le rôle de gélifiant de l'émulsion.

En outre, l'émulsion selon l'invention peut contenir un ou plusieurs sels inorganiques dont la présence va permettre encore une diminution de l'activité en eau de l'émulsion. Comme sels, on peut citer en particulier les sels de magnésium, de calcium et de sodium, et plus spécialement le sulfate de magnésium, le chlorure de magnésium, le chlorure de calcium et le chlorure de sodium. Le ou les sels peuvent être présents en une quantité allant de 0,1 à 30 % et de préférence de 2 à 12 % en poids par rapport au poids total de la composition.

Les actifs sensibles à l'eau qui peuvent être utilisés selon l'invention sont notamment les enzymes (par exemple lactoperoxydase, lipase, protéase, phospholipase, cellulases), les extraits naturels tels que thé vert, extrait de mélisse, extrait de thym, les oligomères procyannidoliques (OPC) tels que l'OPC d'aubépine, l'OPC de pin et l'OPC de raisin, les vitamines et notamment l'acide ascorbique (vitamine C) et ses esters, les esters de rétinol (vitamine A), les dérivés phosphatés et glucosilés, l'urée et la rutine.

Le ou les actifs sensibles à l'eau utilisés sont de manière avantageuse une enzyme, et plus particulièrement une protéase. Cette protéase peut être choisie par exemple parmi celle vendue sous la dénomination commerciale "Subtilisine SP 544" par la société Novo Nordisk et celle vendue sous la dénomination commerciale "Lysoveg" par la société Laboratoires Sérobiologiques de Nancy.

La quantité d'actif sensible à l'eau dans l'émulsion selon l'invention dépend du type d'actif utilisé. De manière générale, le ou les actifs peuvent être utilisés dans l'émulsion selon l'invention en une quantité allant de 0,001 à 15 % en poids, de préférence de 0,01 à 10 % en poids par rapport au poids total de l'émulsion.

La proportion de la phase huileuse dans l'émulsion selon l'invention peut aller de 10 à 40 % en poids, et de préférence de 15 à 30 % en poids par rapport au poids total de l'émulsion.

La phase huileuse contient 50 à 100 % en poids d'organopolysiloxanes, c'est-à-dire d'huiles siliconées ; celles-ci représentent de préférence de 60 à 80 % en poids, et mieux de 65 à 75 % en poids par rapport à la quantité totale de phase huileuse. Ces huiles siliconées peuvent être notamment des silicones volatiles telles que le cyclopentadiméthylsiloxane et le cyclotétradiméthylsiloxane, ou des polydiméthylsiloxanes.

La phase huileuse peut comprendre, en plus des huiles siliconées, des huiles hydrocarbonées et fluorées, ces huiles non siliconées étant présentes en une quantité inférieure à 50 % en poids par rapport au poids total de la phase huileuse. Comme huiles hydrocarbonées utilisables dans l'invention, on peut citer les huiles minérales (huile de vaseline), les huiles végétales (huile de rosier muscat), les huiles animales, les huiles de synthèse. Comme huiles fluorées, on peut citer par exemple les perfluoropolyéthers. On peut aussi utiliser des matières grasses comme les alcools gras, les acides gras (acide stéarique), les cires et les gommes (cires ou gommes de silicone).

L'émulsionnant siliconé selon l'invention est notamment un diméthicone copolyol ayant une valeur de HLB (Hydrophilic Lipophilic Balance) égale ou supérieure à 10, de préférence un alkyldiméthicone copolyol choisi parmi les alkyldiméthicone copolyols en $C_{10}$-$C_{22}$ polyoxyéthylénés et/ou polyoxypropylénés. Il peut s'agir notamment du diméthicone copolyol butyl éther vendu par la société Shin-Etsu sous la dénomination KF355.

Cet émulsionnant peut être en particulier présent en une proportion allant de 1 à 10 % en poids, et de préférence de 2 à 5 % en poids par rapport au poids total de l'émulsion.

Pour une application topique, l'émulsion selon l'invention doit contenir un milieu topiquement acceptable, c'est-à-dire compatible avec la peau et les cheveux, et la composition à base de cette émulsion peut constituer notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils et/ou les sourcils.

Aussi, l'invention a encore pour objet l'utilisation de la composition selon l'invention pour nettoyer et/ou protéger la peau et/ou les fibres kératiniques, c'est-à-dire les cheveux et/ou les cils.

La présente invention a aussi pour objet une composition nettoyante pour la peau et/ou les fibres kératiniques sous forme d'une composition telle que définie ci-dessus.

L'invention a enfin pour objet un procédé cosmétique et/ou dermatologique pour nettoyer et/ou protéger la peau et/ou les fibres kératiniques, caractérisé en ce qu'il consiste à appliquer sur la peau et/ou les fibres kératiniques une composition telle que définie ci-dessus.

La composition selon l'invention peut constituer notamment des crèmes de protection, de traitement ou de soin pour le visage, pour les mains, pour les pieds, pour les cheveux, des laits corporels de protection ou de soin, des lotions ou mousses pour le soin de la peau, des muqueuses, des cheveux et du cuir chevelu.

De façon connue, la composition de l'invention peut contenir également des adjuvants habituels dans les domaines cosmétique et dermatologique, tels que les tensioactifs moussants, les actifs hydrophiles ou lipophiles en plus des actifs sensibles à l'eau, les conservateurs, les antioxydants, les solvants, les parfums, les charges, les filtres, les absorbeurs d'odeur et les matières colorantes. Elle peut comprendre aussi des microcapsules, des microparticules, ou des vésicules lipidiques de type ionique et/ou non ionique. Les quantités de ces différents adjuvants sont celles classiquement utilisées dans les domaines considérés, et par exemple de 0,01 à 10 % du poids total de l'émulsion. Ces adjuvants, selon leur nature, peuvent être introduits dans la phase grasse, dans la phase aqueuse et/ou dans les vésicules lipidiques.

Comme actifs hydrophiles, on peut utiliser par exemple les protéines ou les hydrolysats de protéine, les acides aminés, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles.

Les exemples ci-après de compositions selon l'invention, sont donnés à titre d'illustration et sans caractère limitatif. Les quantités y sont données en % en poids.

**Exemple 1 : Crème de soin**

| Phase aqueuse : | |
|---|---|
| Norgel | 82 % |
| Diméthicone copolyol butyl éther (KF355 vendu par la société Shin-Etsu) (émulsionnant) (HLB = 14,4) | 3 % |
| Subtilisine SP 544 | 0,1 % |

| Phase huileuse : | |
|---|---|
| Huile de rosier muscat | 4,4 % |
| Cyclométhicone | 10,5 % |

L'émulsion est préparée en émulsionnant la phase huileuse dans la phase aqueuse sous agitation à l'homogénéisateur.

L'émulsion obtenue a une activité en eau de 0,64 ± 0,02.

On obtient une crème d'aspect irisé, apte à lisser la peau et à exfolier les cellules mortes.

**Exemple 2 : Crème de soin**

| Phase aqueuse : | |
|---|---|
| Norgel | 68 % |
| Diméthicone copolyol butyl éther (KF355 vendu par la société Shin-Etsu) (émulsionnant) (HLB = 14,4) | 3 % |
| Subtilisine SP 544 | 0,1 % |

| Phase huileuse : | |
|---|---|
| Huile de rosier muscat | 8 % |
| Cyclométhicone | 20,9 % |

L'émulsion est préparée de la même manière que dans l'exemple 1. Elle a une activité en eau de 0,66 ± 0,02.

On obtient une crème blanche apte à lisser la peau.

**Exemple 3 : Crème de soin**

| Phase aqueuse : | |
|---|---|
| Glycérine | 30 % |
| Propylène glycol | 8 % |
| Diméthicone copolyol butyl éther (KF355 vendu par la société Shin-Etsu) (émulsionnant) (HLB = 14,4) | 3 % |
| Chlorure de magnésium anhydre | 2 % |
| Eau | qsp 100 % |

| Phase huileuse : | |
|---|---|
| Huile de rosier muscat | 4,4 % |
| Cyclométhicone | 10,5 % |

L'émulsion est préparée de la même manière que dans l'exemple 1. On obtient une crème blanche apte à hydrater la peau.

**Revendications**

1. Emulsion comprenant une phase huileuse dispersée dans une phase aqueuse à l'aide d'un agent émulsionnant siliconé, dont la phase huileuse comporte au moins 50 % en poids d'huile siliconée, caractérisée en ce que la phase aqueuse contient au moins un polyol en une quantité efficace pour obtenir une valeur d'activité en eau de l'émulsion, inférieure ou égale à 0,85.

2. Emulsion selon la revendication 1, caractérisée en ce que le polyol est présent en une quantité efficace pour obtenir une valeur d'activité en eau de l'émulsion, inférieure ou égale à 0,7.

3. Emulsion selon la revendication 1 ou 2, caractérisée en ce que la phase aqueuse est présente en une quantité allant de 60 à 90 % en poids par rapport au poids total de l'émulsion.

4. Emulsion selon l'une quelconque des revendications précédentes, caractérisée en ce que le polyol est présent en une quantité d'au moins 30 % en poids par rapport au poids total de la composition.

5. Emulsion selon l'une quelconque des revendications précédentes, caractérisée en ce que le polyol est présent en une quantité allant de 40 à 90 % en poids par rapport au poids total de la composition.

6. Emulsion selon l'une quelconque des revendications précédentes, caractérisée en ce que le polyol est choisi dans le groupe comprenant la glycérine et les glycols.

7. Emulsion selon l'une quelconque des revendications précédentes, caractérisée en ce que le polyol est complexé avec un polymère acrylique ou méthacrylique.

8. Emulsion selon la revendication précédente, caractérisée en ce que le polymère comprend en outre de l'eau liée.

9. Emulsion selon l'une quelconque des revendications précédentes, caractérisée en ce que le polymère avec le polyol et l'eau complexés est présent en une quantité allant de 60 à 90 % en poids par rapport au poids total de l'émulsion.

10. Emulsion selon l'une quelconque des revendications précédentes, caractérisée en ce que l'huile siliconée représente de 60 à 80 % en poids de la phase huileuse.

11. Emulsion selon l'une quelconque des revendications précédentes, caractérisée en ce que l'huile siliconée est choisie dans le groupe comprenant les silicones volatiles et les polydiméthylsiloxanes.

12. Emulsion selon l'une quelconque des revendications précédentes, caractérisée en ce que l'émulsionnant a une valeur de HLB supérieure ou égale à 10.

13. Emulsion selon la revendication précédente, caractérisée en ce que l'émulsionnant est un alkyldiméthicone copolyol.

14. Emulsion selon la revendication 12 ou 13, caractérisée en ce que l'émulsionnant est le diméthicone copolyol butyl éther.

15. Emulsion selon l'une quelconque des revendications précédentes, caractérisée en ce que l'émulsionnant est présent en une quantité allant de 1 à 10 % en poids par rapport au poids total de l'émulsion.

16. Emulsion selon l'une quelconque des revendications précédentes, caractérisée en ce qu'elle contient, en outre, au moins un sel inorganique.

17. Emulsion selon la revendication précédente, caractérisée en ce que le sel est choisi dans le groupe comprenant les sels de magnésium, les sels de calcium et les sels de sodium.

18. Emulsion selon la revendication 16 ou 17, caractérisée en ce que le ou les sels sont présents en une quantité allant de 0,1 à 30 % en poids par rapport au poids total de la composition.

19. Composition, caractérisée en ce qu'elle contient au moins un actif à action topique sensible à l'eau et une émulsion selon l'une quelconque des revendications précédentes.

20. Composition selon la revendication précédente, caractérisée en ce que l'actif à action topique sensible à l'eau est choisi dans le groupe comprenant les enzymes, les extraits naturels, les oligomères procyannidoliques, les vitamines, les dérivés phosphatés et glucosilés, l'urée et la rutine.

21. Composition selon la revendication précédente 19 ou 20, caractérisée en ce que l'actif à action topique sensible à l'eau est choisi dans le groupe comprenant une protéase, le thé vert, l'acide ascorbique.

22. Composition selon l'une quelconque des revendications 19 à 21, caractérisée en ce que l'actif sensible à l'eau est présent en une concentration allant de 0,001 à 15 % en poids par rapport au poids total de l'émulsion.

23. Composition selon l'une quelconque des revendications 19 à 22, caractérisée en ce qu'elle constitue une composition cosmétique et/ou dermatologique.

24. Composition selon l'une quelconque des revendications 19 à 23, caractérisée en ce qu'elle comprend au moins un adjuvant lipophile ou hydrophile choisi parmi les conservateurs, les antioxydants, les parfums, les charges, les filtres, les séquestrants, les huiles essentielles, les matières colorantes, les actifs hydrophiles ou lipophiles, et les vésicules lipidiques.

25. Composition nettoyante pour la peau et/ou les fibres kératiniques, caractérisée en ce qu'elle consiste en une composition selon l'une quelconque des revendications 19 à 24.

26. Utilisation de la composition selon l'une quelconque des revendications 19 à 24 pour nettoyer et/ou protéger la peau et/ou les fibres kératiniques.

27. Procédé cosmétique pour nettoyer et/ou protéger la peau et/ou les fibres kératiniques, caractérisé en ce qu'il consiste à appliquer sur la peau et/ou les fibres kératiniques une composition selon l'une quelconque des revendications 19 à 26.

## Claims

1. Emulsion comprising an oily phase dispersed in an aqueous phase with the aid of a silicone emulsifying agent, whose oily phase comprises at least 50 % by weight of silicone oil, characterized in that the aqueous phase contains at least one polyol in an effective quantity to obtain a water activity value of the emulsion of less than or equal to 0.85.

2. Emulsion according to Claim 1, characterized in that the polyol is present in an effective quantity to obtain a water activity value of the emulsion of less than or equal to 0.7.

3. Emulsion according to Claim 1 or 2, characterized in that the aqueous phase is present in a quantity ranging from 60 to 90 % by weight relative to the total weight of the emulsion.

4. Emulsion according to any one of the preceding claims, characterized in that the polyol is present in a quantity of at least 30 % by weight relative to the total weight of the composition.

5. Emulsion according to any one of the preceding claims, characterized in that the polyol is present in a quantity ranging from 40 to 90 % by weight relative to the total weight of the composition.

6. Emulsion according to any one of the preceding claims, characterized in that the polyol is chosen from the group comprising glycerine and glycols.

7. Emulsion according to any one of the preceding claims, characterized in that the polyol is complexed with an acrylic or methacrylic polymer.

8. Emulsion according to the preceding claim, characterized in that the polymer comprises, in addition, bound water.

9. Emulsion according to any one of the preceding claims, characterized in that the polymer with the complexed polyol and water is present in a quantity ranging from 60 to 90 % by weight relative to the total weight of the emulsion.

10. Emulsion according to any one of the preceding claims, characterized in that the silicone oil represents 60 to 80 % by weight of the oily phase.

11. Emulsion according to any one of the preceding claims, characterized in that the silicone oil is chosen from the group comprising volatile silicones and polydimethylsiloxanes.

12. Emulsion according to any one of the preceding claims, characterized in that the emulsifier has an HLB value greater than or equal to 10.

13. Emulsion according to the preceding claim, characterized in that the emulsifier is an alkyldimethicone copolyol.

14. Emulsion according to claim 12 or 13, characterized in that the emulsifier is dimethicone copolyol butyl ether.

15. Emulsion according to any one of the preceding claims, characterized in that the emulsifier is present in a quantity ranging from 1 to 10 % by weight relative to the total weight of the emulsion.

16. Emulsion according to any one of the preceding claims, characterized in that it additionally contains at least one inorganic salt.

17. Emulsion according to the preceding claim, characterized in that the salt is chosen from the group comprising magnesium salts, calcium salts and sodium salts.

18. Emulsion according to Claim 16 or 17, characterized in that the salt(s) is/are present in a quantity ranging from 0.1 to 30 % by weight relative to the total weight of the composition.

19. Composition, characterized in that it contains at least one water-sensitive active agent with topical action and an emulsion according to any one of the preceding claims.

20. Composition according to the preceding claim, characterized in that the water-sensitive active agent with topical action is chosen from the group comprising enzymes, natural extracts, procyannidolic oligomers, vitamins, phosphated and glucosylated derivatives, urea and rutin.

21. Composition according to the preceding Claim 19 or 20, characterized in that the water-sensitive active agent with topical action is chosen from the group comprising a protease, green tea and ascorbic acid.

22. Composition according to any one of Claims 19 to 21, characterized in that the water-sensitive active agent is present in a concentration ranging from 0.001 to 15 % by weight relative to the total weight of the emulsion.

23. Composition according to any one of Claims 19 to 22, characterized in that it constitutes a cosmetic and/or dermatological composition.

24. Composition according to any one of Claims 19 to 23, characterized in that it comprises at least one lipophilic or hydrophilic adjuvant chosen from preservatives, antioxidants, perfumes, fillers, screening agents, sequestrants, essential oils, colouring matter, hydrophilic or lipophilic active agents and lipid vesicles.

25. Cleansing composition for the skin and/or the keratinous fibres, characterized in that it consists of a composition according to any one of Claims 19 to 24.

26. Use of the composition according to any one of Claims 19 to 24 for cleansing and/or protecting the skin and/or the keratinous fibres.

27. Cosmetic process for cleansing and/or protecting the skin and/or the keratinous fibres, characterized in that it consists in applying to the skin and/or the keratinous fibres a composition according to any one of Claims 19 to 26.

**Patentansprüche**

1. Emulsion, die eine durch einen Siliconemulgator in einer wäßrigen Phase dispergierte Ölphase enthält, wobei die Ölphase mindestens 50 Gew.-% Siliconöl aufweist,
dadurch gekennzeichnet, daß
die wäßrige Phase mindestens ein Polyol in einem Mengenanteil enthält, der wirksam ist, einen Wert der Wasseraktivität der Emulsion von höchstens 0,85 einzustellen.

2. Emulsion nach Anspruch 1, dadurch gekennzeichnet, daß das Polyol in einem Mengenanteil vorliegt, der wirksam ist, um einen Wert der Wasseraktivität der Emulsion von höchstens 0,7 einzustellen.

3. Emulsion nach Anspruch 1 oder 2, dadurch gekennzeichnet, daß die wäßrige Phase in einem Mengenanteil von 60 bis 90 Gew.-%, bezogen auf das Gesamtgewicht der Emulsion, vorliegt.

4. Emulsion nach einem der vorhergehenden Ansprüche, dadurch gekennzeichnet, daß das Polyol in einem Mengenanteil von mindestens 30 Gew.-%, bezogen auf das Gesamtgewicht der Zusammensetzung, vorliegt.

5. Emulsion nach einem der vorhergehenden Ansprüche, dadurch gekennzeichnet, daß das Polyol in einem Mengenanteil von 40 bis 90 Gew.-%, bezogen auf das Gesamtgewicht der Zusammensetzung, vorliegt.

6. Emulsion nach einem der vorhergehenden Ansprüche, dadurch gekennzeichnet, daß das Polyol unter Glycerin und den Glykolen ausgewählt ist.

7. Emulsion nach einem der vorhergehenden Ansprüche, dadurch gekennzeichnet, daß das Polyol mit einem Acryl- oder Methacrylpolymer komplexiert ist.

8. Emulsion nach dem vorhergehenden Anspruch, dadurch gekennzeichnet, daß das Polymer ferner gebundenes Wasser enthält.

9. Emulsion nach einem der vorhergehenden Ansprüche, dadurch gekennzeichnet, daß das mit dem Polyol und Wasser komplexierte Polymer in einem Mengenanteil von 60 bis 90 Gew.-%, bezogen auf das Gesamtgewicht der Emulsion, vorliegt.

10. Emulsion nach einem der vorhergehenden Ansprüche, dadurch gekennzeichnet, daß das Siliconöl 60 bis 80 % der Ölphase ausmacht.

11. Emulsion nach einem der vorhergehenden Ansprüche, dadurch gekennzeichnet, daß das Siliconöl unter den flüchtigen Siliconen und Polydimethylsiloxanen ausgewählt ist.

12. Emulsion nach einem der vorhergehenden Ansprüche, dadurch gekennzeichnet, daß der Emulgator einen HLB-Wert von mindestens 10 aufweist.

13. Emulsion nach dem vorhergehenden Anspruch, dadurch gekennzeichnet, daß der Emulgator ein Alkyldimethiconcopolyol ist.

14. Emulsion nach Anspruch 12 oder 13, dadurch gekennzeichnet, daß der Emulgator der Dimethiconcopolyolbutylether ist.

15. Emulsion nach einem der vorhergehenden Ansprüche, dadurch gekennzeichnet, daß der Emulgator in einem Mengenanteil von 1 bis 10 Gew.-%, bezogen auf das Gesamtgewicht der Emulsion, vorliegt.

**16.** Emulsion nach einem der vorhergehenden Ansprüche, dadurch gekennzeichnet, daß sie ferner mindestens ein anorganisches Salz enthält.

**17.** Emulsion nach dem vorhergehenden Anspruch, dadurch gekennzeichnet, daß das Salz unter den Salzen von Magnesium, Calcium und Natrium ausgewählt ist.

**18.** Emulsion nach Anspruch 16 oder 17, dadurch gekennzeichnet, daß das Salz oder die Salze in einem Mengenanteil von 0,1 bis 30 Gew.-%, bezogen auf das Gesamtgewicht der Zusammensetzung, vorliegen.

**19.** Zusammensetzung, dadurch gekennzeichnet, daß sie mindestens einen gegenüber Wasser empfindlichen Wirkstoff zur topischen Einwirkung und eine Emulsion nach einem der vorhergehenden Ansprüche enthält.

**20.** Zusammensetzung nach dem vorhergehenden Anspruch, dadurch gekennzeichnet, daß der wasserempfindliche Wirkstoff zur topischen Einwirkung unter den Enzymen, natürlichen Extrakten, Procyanidinoligomeren, Vitaminen, Phosphat- und Glucosylderivaten, Harnstoff und Rutin ausgewählt ist.

**21.** Zusammensetzung nach Anspruch 19 oder 20, dadurch gekennzeichnet, daß der gegenüber Wasser empfindliche Wirkstoff zur topischen Einwirkung unter einer Protease, grünem Tee und Ascorbinsäure ausgewählt ist.

**22.** Zusammensetzung nach einem der Ansprüche 19 bis 21, dadurch gekennzeichnet, daß der gegenüber Wasser empfindliche Wirkstoff in einer Konzentration von 0,001 bis 15 Gew.-%, bezogen auf das Gesamtgewicht der Emulsion, vorliegt.

**23.** Zusammensetzung nach einem der Ansprüche 19 bis 22, dadurch gekennzeichnet, daß sie eine kosmetische und/oder dermatologische Zusammensetzung ist.

**24.** Zusammensetzung nach einem der Ansprüche 19 bis 23, dadurch gekennzeichnet, daß sie mindestens einen lipophilen oder hydrophilen Hilfsstoff enthält, der unter den Konservierungsmitteln, Antioxidantien, Parfums, Füllstoffen, Filtern, Maskierungsmitteln, etherischen Ölen, Färbemitteln, hydrophilen oder lipophilen Wirkstoffen und Lipidvesikeln ausgewählt ist.

**25.** Zusammensetzung zur Reinigung der Haut und/oder von Keratinfasern, dadurch gekennzeichnet, daß sie aus einer Zusammensetzung nach einem der Ansprüche 19 bis 24 besteht.

**26.** Verwendung der Zusammensetzung nach einem der Ansprüche 19 bis 24 zur Reinigung und/oder zum Schutz der Haut und/oder von Keratinfasern.

**27.** Kosmetisches Verfahren zur Reinigung und/oder zum Schutz der Haut und/oder von Keratinfasern, dadurch gekennzeichnet, daß es darin besteht, auf die Haut und/oder die Keratinfasern eine Zusammensetzung nach einem der Ansprüche 19 bis 26 aufzutragen.